# EUROPEAN PATENT APPLICATION

(11) **EP 4 702 989 A1**
(43) Date of publication of application: **04.03.2026**
(21) Application number: 24796206.1
(22) Date of filing: 26.04.2024
(51) Int. Cl.: A61K 39/395, A61K 31/4406, A61P 35/00, A61P 35/04

(54) **DRUG FOR TREATING LOCALLY ADVANCED UNRESECTABLE OR METASTATIC COLORECTAL CANCER AND USE THEREOF**

(30) Priority: 27.04.2023 CN 202310481472; 08.03.2024 CN 202410263692
(71) Applicant: Shenzhen Chipscreen Biosciences Co., Ltd., Guangdong 518057 (CN)
(72) Inventor: LU, Xianping, Shenzhen, Guangdong 518057 (CN); PAN, Desi, Shenzhen, Guangdong 518057 (CN); WANG, Xiaoning, Shenzhen, Guangdong 518057 (CN)
(74) Representative: Schnappauf, Georg
(86) International application number: PCT/CN2024/089938
(87) International publication number: WO 2024/222832

(57) **Abstract**

A drug for treating locally advanced unresectable or metastatic colorectal cancer and a use thereof. Compared with the solution of a two-drug combination of Sintilimab + Chidamide, the provided solution of a three-drug combination of Sintilimab + Chidamide + IBI305 can significantly prolong the progress-free survival of a patient with MSS/MSI-L locally advanced unresectable or metastatic colorectal cancer. Additionally, in terms of ORR, PR, PD, NE, DCR, etc., the three-drug combination of Sintilimab + Chidamide + IBI305 can also significantly benefit the patient with MSS/MSI-L locally advanced unresectable or metastatic colorectal cancer.

## Description

### TECHNICAL FIELD

The present invention relates to the field of pharmaceuticals, and in particular to a medicament for treatment a locally advanced unresectable or metastatic colorectal cancer and uses thereof.

### BACKGROUND ART

Colorectal cancer refers to malignant changes in the epithelial cells of the large intestinal mucosa, including abnormal differentiation, abnormal proliferation, and abnormalities in cell metabolism and secretion, etc. The vast majority (> 95%) of colorectal cancers in clinical practice are adenocarcinomas, with 1% being squamous cell carcinomas. Intestinal cancer cells can metastasize to other parts of the body, such as the liver, lungs, and brain, via the blood circulation, or spread to adjacent lymph nodes or glands via the lymphatic circulation.

In recent years, with the continuous improvement of people's living standards and changes in dietary structure, a dietary structure that is high in protein and fat and low in fiber has led to a year-by-year increase in the incidence of colorectal cancer. Between 1972 and 1989 in China, the incidence of colorectal cancer increased by 79% among both males and females. Since the 1970s, it has grown at an annual rate of 4.2%. In the cancer spectrum in China, the colorectal cancer ranks fifth, following gastric cancer, liver cancer, lung cancer, and esophageal cancer. Statistics show that the incidence of colorectal cancer in China was less than 10 per 100,000 in the 1970s, but rose to 23.4 per 100,000 in the early 1990s.

Early-stage colorectal cancer is primarily treated with surgical resection, and the 5-year survival rate after surgery for early-stage cancer can exceed 90%. However, approximately 25% of colorectal cancer patients already have metastasis at the time of initial diagnosis. In addition, up to 50% of newly diagnosed patients will eventually progress to metastatic colorectal cancer, with less than 5% of those with metastases surviving beyond five years. The most common sites of metastasis are the liver or nearby lymph nodes. The medical demands for metastatic colorectal cancer are very high.

Currently, the treatment of advanced colorectal cancer primarily relies on pharmacological treatment. It includes, but is not limited to, standard first-line treatment involving FOLFOX/FOLFIRI combined with targeted treatment. After disease progression, it includes, but is not limited to, a standard second-line treatment regimen combining FOLFIRI + bevacizumab. However, the overall response rate of second-line treatment is low, with short progression-free survival and overall survival. Therefore, new treatment regimens still need to be further developed.

Chidamide is a Class 1.1 new drug developed by Shenzhen Chipscreen Biosciences Co., Ltd. It is a benzamide histone deacetylase (HDAC) subtype-selective inhibitor targeting HDAC subtypes 1, 2, and 3 of Class I and subtype 10 of Class IIb, exhibiting regulatory effects on abnormal tumor epigenetic functions. Currently approved indications for chidamide include: 1) peripheral T-cell lymphoma; 2) relapsed or progressed locally advanced or metastatic breast cancer in hormone receptor-positive and human epidermal growth factor receptor-2 negative postmenopausal patients after an endocrine therapy.

CN03139760.3 discloses a chidamide compound, in particular benzamide histone deacetylase inhibitors with differentiation and antiproliferative activities, as well as a preparation method and uses of their pharmaceutical preparations. It discloses a general structural formula and defines the substituents. These compounds, as histone deacetylase inhibitors, can be used for treating diseases related to differentiation and proliferation, such as cancer and psoriasis.

CN201210489178.8 discloses two crystalline forms of chidamide, namely chidamide crystal form A and chidamide crystal form B, as well as preparation methods for the new crystal forms of chidamide. The crystal form A of chidamide and the crystal form B of chidamide exhibit excellent performance in terms of oral absorption and inhibition of cell differentiation and proliferation, with low toxicity and good stability in storage and handling, and they can be used in the preparation of medicaments for treating diseases related to cell differentiation and proliferation.

CN201410136761.X discloses an E-configuration benzamide compound, and a pharmaceutical preparations and uses thereof. The E-configuration benzamide compound is chidamide, with the chemical name N-(2-amino-4-fluorophenyl)-4-[N-[(E)-3-(3-pyridyl)acryloyl]aminomethyl]benzamide, wherein the configuration of the 3-pyridylacryloyl group in its structural formula is E-type. The E-configuration chidamide exhibits a subtype-selective inhibitory activity against histone deacetylases, primarily against HDAC1, HDAC2, and HDAC3 among Class I HDACs, as well as HDAC10 among Class IIb HDACs. The E-configuration chidamide can be used to treat diseases associated with abnormal histone deacetylase activity, such as cancers, including lymphoma, solid tumors, and hematological malignancies.

Sintilimab is a PD-1 inhibitor jointly developed by Innovent Biologics and Eli Lilly in China. It is a human immunoglobulin G4 (IgG4) monoclonal antibody capable of specifically binding to PD-1 molecules on the surface of T cells, thereby blocking the PD-1/programmed death-ligand 1 (PD-L1) pathway responsible for tumor immune tolerance. This reactivates the anti-tumor activity of lymphocytes, achieving the goal of treating tumors. Currently approved indications for sintilimab include classical Hodgkin's lymphoma, first-line non-squamous non-small cell lung cancer, first-line squamous cell lung carcinoma, and first-line hepatocellular carcinoma.

Bevacizumab is a humanized monoclonal antibody IgG1 originally developed by Roche using the recombinant DNA technology. It plays a significant role in tumor growth and proliferation by inhibiting the vascular endothelial growth factor (VEGF). The VEGF family includes multiple related factors such as VEGF-A, VEGF-B, VEGF-C, and VEGF-D, among which VEGF-A plays the most critical role in tumor angiogenesis by promoting the growth and proliferation of vascular endothelial cells and binding to growth factor receptors produced by vascular endothelial cells to activate downstream signal transduction pathways, ultimately facilitating the formation of new blood vessels. Bevacizumab was first approved in the United States in 2004 for the treatment of advanced colorectal cancer, and became the first widely used anti-angiogenic therapy for patients with advanced cancer. It was marketed under the brand name Avastin and was first introduced in China in 2010. Avastin has been approved for six indications in China, including: 1) metastatic colorectal cancer (treated in combination with chemotherapies); 2) unresectable advanced, metastatic, or recurrent non-squamous non-small cell lung cancer (in the first-line treatment); 3) recurrent glioblastoma; 4) unresectable hepatocellular carcinoma in patients who have not received a previous systemic therapy (treated in combination with atezolizumab); 5) advanced epithelial ovarian cancer, fallopian tube cancer, or primary peritoneal cancer in patients (treated in combination with carboplatin and paclitaxel for the first-line treatment); and 6) persistent, recurrent, or metastatic cervical cancer in patients (treated in combination with paclitaxel and cisplatin or paclitaxel and topotecan). As of June 2022, there were 9 bevacizumab products available in China, including: Avastin (Roche), Anda (Qilu), IBI305/Byvasda (Innovent), Beianting (MIL60, Betta), Airuituo (Hengrui), Hanbeitai (HLX04), Pusintin, Pubeixi, and Boyunuo. MMR gene is a DNA mismatch repair gene and has the expressions of four common MMR proteins (including MLH1, PMS2, MSH2, and MSH6) detected by immunohistochemical methods, with the positive expression localized in the cell nucleus. The deficiency of its expression can lead to the accumulation of mismatches during DNA replication, resulting in microsatellite instability (MSI). Approximately 15% of colorectal cancers are initiated through the MSI pathway. dMMR indicates deficient MMR expression, while pMMR indicates proficient MMR expression without deficiency. dMMR exhibits high-frequency microsatellite instability (MSI-H), while pMMR exhibits low-frequency microsatellite instability (MSI-L) or microsatellite stability (MSS).

### SUMMARY OF THE INVENTION

An object of the present invention is to provide a medicament for treatment of a locally advanced unresectable or metastatic colorectal cancer and uses thereof.

In the first aspect, the present invention provides use of a triple-drug combination of a component (1), a component (2), and a component (3) in first-line, second-line or third-line and higher-line prevention and/or treatment and/or amelioration of a locally advanced unresectable or metastatic colorectal cancer disease or disease progression, wherein the component (1), the component (2), and the component (3) are each as defined herein.

In the second aspect, the present invention provides use of a triple-drug combination of a component (1), a component (2), and a component (3) in preparation of a medicament for first-line, second-line or third-line and higher-line prevention and/or treatment and/or amelioration of a locally advanced unresectable or metastatic colorectal cancer disease or disease progression, wherein the component (1), the component (2), and the component (3) are each as defined herein.

In the third aspect, the present invention provides a pharmaceutical composition for first-line, second-line or third-line and higher-line prevention and/or treatment and/or amelioration of a locally advanced unresectable or metastatic colorectal cancer disease or disease progression, comprising a component (1), a component (2), and a component (3), wherein the component (1), the component (2), and the component (3) are each as defined herein.

In the fourth aspect, the present invention provides the pharmaceutical composition as described above for use in first-line, second-line or third-line and higher-line prevention and/or treatment and/or amelioration of the locally advanced unresectable or metastatic colorectal cancer disease or disease progression.

In the fifth aspect, the present application provides a medication cassette, comprising the pharmaceutical composition as described above.

In a specific embodiment, in the medication cassette as described above, the component (1), the component (2), and the component (3) are unit preparations having the same or different strengths, and the component (1), the component (2), and the component (3) are placed in the same container or in separate containers respectively.

In a specific embodiment, in the medication cassette as described above, the component (1) is a gastrointestinal dosage form, preferably an oral preparation; and the component (2) and the component (3) are parenteral dosage forms, preferably injectable preparations.

In a specific embodiment, the medication cassette as described above further comprises a label containing the following information: a component (1), to be administered twice weekly starting from Day 1 of Cycle 1, with an interval of no less than 3 days between doses; a component (2), to be administered on Day 1 of each 3-week treatment cycle; and a component (3), to be administered on Day 1 of each 3-week treatment cycle.

In a specific embodiment, in the medication cassette as described above, the label further comprises the following information: a component (1) of 30 mg, to be administered twice weekly via PO 30 minutes after meals, starting from Day 1 of Cycle 1, with an interval of no less than 3 days between doses.

In a specific embodiment, in the medication cassette as described above, the label further comprises the following information: a component (2) of 200 mg, to be administered via IV drip on Day 1 of each three-week treatment cycle.

In a specific embodiment, in the medication cassette as described above, the label further comprises the following information: a component (3) of 7.5 mg/kg, to be administered via IV drip on Day 1 of each three-week treatment cycle.

In a specific embodiment, in the medication cassette as described above, the label further comprises the following information: administering the component (3) at least 5 minutes after administering the component (2).

In the sixth aspect, the present invention provides a method for first-line, second-line or third-line and higher-line prevention and/or treatment and/or amelioration of a locally advanced unresectable or metastatic colorectal cancer, comprising administering to a patient in need thereof a prophylactically and/or therapeutically and/or amelioratively effective amount of the pharmaceutical composition as described above.

In a specific embodiment, the component (1) in the present invention is selected from the group consisting of chidamide, a pharmaceutically acceptable salt, crystal form, isomer, prodrug, and metabolite thereof.

In a specific embodiment, the component (1) in the present invention is selected from the group consisting of chidamide, a pharmaceutically acceptable salt, and crystal form thereof.

In a specific embodiment, the component (1) in the present invention is selected from the group consisting of chidamide, and crystal forms A and B thereof.

In a specific embodiment, the component (1) in the present invention is chidamide.

In a specific embodiment, the component (2) in the present invention is selected from the group consisting of sintilimab, an antigen-binding fragment thereof, a variant thereof, anda biosimilar thereof.

In a specific embodiment, the component (2) in the present invention is selected from the group consisting of sintilimab, an antigen-binding fragment thereof, and a biosimilar thereof.

In a specific embodiment, the component (2) in the present invention is selected from the group consisting of sintilimaband a biosimilar thereof.

In a specific embodiment, the component (2) in the present invention is selected from sintilimab.

In a specific embodiment, the component (3) in the present invention is selected from the group consisting of bevacizumab, an antigen-binding fragment thereof, a variant thereof , and a biosimilar thereof.

In a specific embodiment, the component (3) in the present invention is selected from the group consisting of bevacizumab, an antigen-binding fragment thereof, and a biosimilar thereof.

In a specific embodiment, the component (3) in the present invention is selected from the group consisting of bevacizumab and a biosimilar thereof.

In a specific embodiment, the component (3) in the present invention is selected from the group consisting of bevacizumab and IBI305.

In a specific embodiment, the component (3) in the present invention is selected from bevacizumab.

In a specific embodiment, the component (3) in the present invention is selected from IBI305.

In a specific embodiment, in the medication cassette as described above, the component (1) is chidamide, with a strength of 5 mg/tablet.

In a specific embodiment, in the medication cassette as described above, the component (2) is sintilimab, with a strength of 100 mg/10 ml;

In a specific embodiment, in the medication cassette as described above, the component (3) as bevacizumab, with a strength of 100 mg/4 ml or 400 mg/16 ml.

In a specific embodiment, in the medication cassette as described above, the component (3) is IBI305, with a strength of 100 mg/4 ml or 400 mg/16 ml.

In a specific embodiment, in the method for first-line, second-line or third-line and higher-line prevention and/or treatment and/or amelioration of a locally advanced unresectable or metastatic colorectal cancer as described above, the component (1), the component (2), and the component (3) are administered simultaneously, separately, or sequentially.

In a specific embodiment, in the method for first-line, second-line or third-line and higher-line prevention and/or treatment and/or amelioration of a locally advanced unresectable or metastatic colorectal cancer as described above, the component (1) is administered 1-4 times per week, preferably twice per week; the component (2) is administered once every 2-5 weeks, preferably once every 3 weeks; and the component (3) is administered once every 2-5 weeks, preferably once every 3 weeks.

In a specific embodiment, in the method for first-line, second-line or third-line and higher-line prevention and/or treatment and/or amelioration of a locally advanced unresectable or metastatic colorectal cancer as described above, the component (1) is administered at a dose of 10-60 mg each time, preferably 20-40 mg each time, and more preferably 30 mg each time.

In a specific embodiment, in the method for first-line, second-line or third-line and higher-line prevention and/or treatment and/or amelioration of a locally advanced unresectable or metastatic colorectal cancer as described above, the component (2) is administered at a dose of 100-300 mg each time, preferably 200 mg each time.

In a specific embodiment, in the method for first-line, second-line or third-line and higher-line prevention and/or treatment and/or amelioration of a locally advanced unresectable or metastatic colorectal cancer as described above, the component (3) is administered at a dose of 5-10 mg/Kg each time, preferably 6-8 mg/Kg each time, and more preferably 7.5 mg/Kg each time.

In a specific embodiment, in the method for first-line, second-line or third-line and higher-line prevention and/or treatment and/or amelioration of a locally advanced unresectable or metastatic colorectal cancer as described above, the component (2) is sintilimab or a biosimilar thereof, preferably sintilimab, and is administered on Day 1 of each treatment cycle of 2-4 weeks, preferably 3 weeks.

In a specific embodiment, in the method for first-line, second-line or third-line and higher-line prevention and/or treatment and/or amelioration of a locally advanced unresectable or metastatic colorectal cancer as described above, the component (3) is bevacizumab or a biosimilar thereof, preferably bevacizumab or IBI305, and is administered on Day 1 of each treatment cycle of 2-4 weeks, preferably 3 weeks.

In a specific embodiment, in the method for first-line, second-line or third-line and higher-line prevention and/or treatment and/or amelioration of a locally advanced unresectable or metastatic colorectal cancer as described above, the component (1) is chidamide, and is orally administered twice weekly 30 minutes after meals, starting from Day 1 of Cycle 1, with an interval of no less than 3 days between doses.

In a specific embodiment, in the method for first-line, second-line or third-line and higher-line prevention and/or treatment and/or amelioration of a locally advanced unresectable or metastatic colorectal cancer as described above, the component (3) is administered at least 5 minutes after administering the component (2), with the component (2) being preferably sintilimab and the component (3) being preferably bevacizumab or IBI305.

In a specific embodiment, in the method for first-line, second-line or third-line and higher-line prevention and/or treatment and/or amelioration of a locally advanced unresectable or metastatic colorectal cancer as described above, the component (2) and the component (3) are both injections administered via intravenous drip; and the component (1) is a tablet administered orally.

In a specific embodiment, the locally advanced unresectable or metastatic colorectal cancer as described in the present invention is of a microsatellite stability (MSS) type or low microsatellite instability (MSI-L) type locally advanced unresectable or metastatic colorectal cancer.

In a specific embodiment, the locally advanced unresectable or metastatic colorectal cancer as described in the present invention is an MSS type locally advanced unresectable or metastatic colorectal cancer.

In a specific embodiment, the locally advanced unresectable or metastatic colorectal cancer as described in the present invention is an MSI-L type locally advanced unresectable or metastatic colorectal cancer.

In a specific embodiment, the locally advanced unresectable or metastatic colorectal cancer as described in the present invention is a locally advanced unresectable or metastatic colorectal adenocarcinoma.

In a specific embodiment, the colorectal adenocarcinoma in the present invention is of a non-adenosquamous-mixed carcinoma type.

In a specific embodiment, the colorectal cancer in the present invention has at least previously undergone failed second-line and higher-line standard treatment or intolerance to toxicity of the second-line and higher-line standard treatment.

In a specific embodiment, the colorectal cancer as described in the present invention is an MSS type or MSI-L type colorectal cancer that has at least previously undergone failed second-line and higher-line standard treatment or intolerance to toxicity of second-line and higher-line standard treatment.

In a specific embodiment, the standard treatment comprises fluorouracils, oxaliplatin, or irinotecan, the fluorouracils comprising 5-fluorouracil, capecitabine, tegafur, and the like. In a further aspect, the present invention relates to use of chidamide in combination with a PD-1 monoclonal antibody and a VEGF monoclonal antibody in preparation of a medicament for treatment of a locally advanced unresectable or metastatic colorectal cancer, wherein:
the colorectal cancer is an MSS type or MSI-L type colorectal cancer that has at least previously undergone failed second-line and higher-line standard treatment or intolerance to toxicity of second-line and higher-line standard treatment.

In a specific embodiment, the standard treatment comprises fluorouracils, oxaliplatin, or irinotecan, the fluorouracils comprising 5-fluorouracil, capecitabine, tegafur, and the like. In a specific embodiment, the colorectal cancer is colorectal adenocarcinoma.

In a specific embodiment, the PD-1 monoclonal antibody comprises nivolumab, pembrolizumab, sintilimab, toripalimab, camrelizumab, tislelizumab, penpulimab, zimberelimab, serplulimab, pucotenlimab, and biosimilars thereof, and the VEGF monoclonal antibody comprises bevacizumab and a biosimilar thereof.

In a further aspect, the present invention relates to a pharmaceutical composition for treatment of a locally advanced unresectable or metastatic colorectal cancer, comprising chidamide, a PD-1 monoclonal antibody, and a VEGF monoclonal antibody, wherein:
the colorectal cancer is an MSS type or MSI-L type colorectal cancer that has at least previously undergone failed second-line and higher-line standard treatment or intolerance to toxicity of second-line and higher-line standard treatment.

In a specific embodiment, the standard treatment comprises fluorouracils, oxaliplatin, or irinotecan, the fluorouracils comprising 5-fluorouracil, capecitabine,or tegafur.

In a specific embodiment, the colorectal cancer is colorectal adenocarcinoma.

In a specific embodiment, the colorectal adenocarcinoma in the present invention is of a non-adenosquamous-mixed carcinoma type.

In a specific embodiment, the PD-1 monoclonal antibody comprises nivolumab, pembrolizumab, sintilimab, toripalimab, camrelizumab, tislelizumab, penpulimab, zimberelimab, serplulimab, pucotenlimab, and biosimilars thereof, and the VEGF monoclonal antibody comprises bevacizumab and a biosimilar thereof.

In a further aspect, the present invention relates to a medication cassette, comprising the pharmaceutical composition as described previously.

In a further aspect, the present invention relates to a method for treatment of a locally advanced unresectable or metastatic colorectal cancer, comprising administering to a patient in need thereof an effective amount of the aforementioned pharmaceutical composition or medication cassette.

In a specific embodiment, the standard treatment comprises fluorouracils, oxaliplatin, or irinotecan, the fluorouracils comprising 5-fluorouracil, capecitabine, or tegafur.

In a specific embodiment, the colorectal cancer is colorectal adenocarcinoma.

In a specific embodiment, the chidamide is administered at a dose of 10-60 mg each time, preferably 10-40 mg each time, and more preferably 30 mg each time; the PD-1 monoclonal antibody is administered at a dose of 100-300 mg each time, preferably 200 mg each time; and the VEGF monoclonal antibody is administered at a dose of 5-10 mg/Kg each time, preferably 6-8 mg/Kg each time, and more preferably 7.5 mg/Kg each time.

In a specific embodiment, the colorectal cancer as described in the present invention has not been previously treated with a small-molecule anti-angiogenic medicament.

In the present invention, "treatment" refers to the administration of a medicament or an additional adjunctive therapy to a subject to achieve a desired pharmacological and/or physiological effect. Based on complete or partial prevention of a disease or its symptoms, this effect may be prophylactic; and/or based on partial or complete stabilization or cure of a disease and/or side effects caused by the disease, this effect may be therapeutic. The term "treatment" as used herein encompasses any treatment of a disease in a patient, comprising: (a) preventing a disease or symptoms in a patient who is predisposed to the disease or symptoms but has not yet been diagnosed as having the same; (b) inhibiting the symptoms of a disease, i.e., preventing its progression; or (c) relieving the symptoms of a disease, i.e., causing the regression of the disease or symptoms.

In the present application, "effective amount" refers to an amount for effectively achieving a desired therapeutic or prophylactic effect at a necessary dose and time. The "therapeutically effective amount" of the pharmaceutical composition of the present invention may vary depending on factors such as the disease state, age, gender, and weight of an individual, as well as the ability of the substance/molecule to elicit a desired response in the individual. The therapeutically effective amount also encompasses an amount of the substance/molecule for achieving a therapeutic beneficial effect that outweighs any toxic or harmful consequences. The "prophylactically effective amount" refers to an amount for effectively achieving a desired prophylactic effect at a necessary dose and duration. Generally but not necessarily, since a prophylactic dose is administered to a subject before the onset of a disease or in the early stage of a disease, the prophylactically effective amount will be less than the therapeutically effective amount. In the case of cancer, the therapeutically effective amount of a medicament can reduce the number of cancer cells; reduce the tumor size; inhibit (i.e., delay to a certain extent, preferably terminate) the infiltration of cancer cells into peripheral organs; inhibit (i.e., delay to a certain extent, preferably terminate) tumor metastasis; inhibit the tumor growth to a certain extent; and/or alleviate one or more symptoms associated with the cancer to some extent.

### BRIEF DESCRIPTION OF THE DRAWING

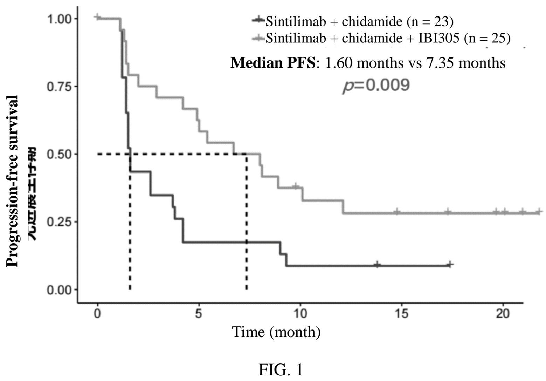

FIG. 1 shows the comparison of PFS between a CAP triple-drug combination regimen and a CP dual-drug combination regimen in the treatment of patients with MSS/MSI-L locally advanced unresectable or metastatic colorectal cancer.

### Beneficial effects of the present invention:

The triple-drug combination regimen of sintilimab + chidamide + IBI305 provided by the present invention can significantly prolong the progression-free survival of patients with MSS/MSI-L locally advanced unresectable or metastatic colorectal cancer as compared to the dual-drug combination regimen of sintilimab + chidamide. In addition, the triple-drug combination regimen of sintilimab + chidamide + IBI305 also demonstrates significant benefits in terms of ORR, PR, PD, NE, DCR and the like for the patients with MSS/MSI-L locally advanced unresectable or metastatic colorectal cancer. Further, with reference to similar types of marketed medicaments, patients that have received the CAP triple-drug combination regimen of the present invention have achieved unexpected and encouraging therapeutic effects in terms of median progression-free survival (PFS), ORR, and the like.

### DETAILED DESCRIPTION OF THE PREFERRED EMBODIMENTS

The present invention discloses a medicament for treatment of an MSS/MSI-L locally advanced unresectable or metastatic colorectal cancer and uses thereof. A person skilled in the art may make appropriate substitutions or modifications by referring to the disclosure herein. It should be particularly noted all similar substitutions and modifications are obvious to a person skilled in the art and should be deemed as being included in the present invention. The medicament for treatment of an MSS/MSI-L locally advanced unresectable or metastatic colorectal cancer and uses thereof according to the present invention have been described by means of preferred embodiments. Obviously, relevant persons can implement and apply the present invention by making modifications or appropriate changes and combinations to the uses and pharmaceutical composition described herein without departing from the content, spirit and scope of the present invention.

### Example 1 Phase II Clinical Study of CAP Triple Regimen versus CP Dual Regimen in Treatment of MSS/MSI-L Type Locally Advanced Unresectable or Metastatic Colorectal Adenocarcinoma

CAP triple-drug regimen: the triple-drug combination regimen of sintilimab + chidamide + IBI305.
CP dual-drug regimen: the dual-drug combination regimen of sintilimab + chidamide.

### I. Test objective and methodology:

This study was a Phase II clinical study taking 18-week progression-free survival (18wPFS) after treatment as a primary endpoint, and objective response rate (ORR), PFS, overall survival (OS), disease control rate (DCR), duration of response (DOR), and safety as secondary endpoints. It was intended to investigate the efficacy and safety of the CP dual-drug regimen and the CAP triple-drug -drug regimen in patients with microsatellite stability type advanced colorectal cancer who had undergone failed standard treatment.

### II. Test subjects:

### Inclusion criteria:

1. Locally advanced unresectable or metastatic colorectal adenocarcinoma (excluding adenosquamous-mixed carcinoma type and other pathological types) confirmed by histopathological or cytological examination.
2. MSS type or MSI-L type confirmed by PCR testing, or proficient mismatch repair (pMMR) confirmed by immunohistochemical detection of the expressions of DNA mismatch repair (MMR) proteins, including MLH1, MSH2, MSH6, and PMS2 proteins, with results showing no protein deficiency.
3. At least previously failed second-line and higher-line standard treatment or intolerance to its toxicity, the standard treatment including fluorouracils (including 5-fluorouracil, capecitabine, tegafur, etc.), oxaliplatin, or irinotecan.
4. At least one measurable lesion according to RECIST v1.1.
5. ECOG PS score of 0-1.
6. Age of no less than 18 years old and no more than 75 years old.
7. Adequate organ and bone marrow functions, with laboratory test values meeting the following requirements within 7 days prior to enrollment, and no treatment with chemotherapy or immunotherapy, or no other treatment contraindications.
   1) Complete blood count: absolute neutrophil count (ANC) ≥ 1.5 × 10⁹/L; platelet count (PLT) ≥ 100 × 10⁹/L; hemoglobin (HGB) ≥ 9.0 g/dL.
   2) Liver function: serum total bilirubin (TBIL) ≤ 1.5 × upper limit of normal (ULN); alanine aminotransferase (ALT) and aspartate aminotransferase (AST) ≤ 3.0 × ULN for subjects without liver metastases, and ALT and AST ≤ 5.0 × ULN for subjects with liver metastases; serum albumin ≥ 25 g/L.
   3) Renal function: serum creatinine (Cr) ≤1.5 × ULN.
   4) Urinalysis: results showed urine protein < 2+; and for patients with baseline urinalysis showing urine protein ≥ 2+, a 24-hour urine collection should be performed with 24-hour urine protein quantification of < 1 g.
   5) Coagulation function: international normalized ratio (INR) ≤ 1.5 × ULN, and activated partial thromboplastin time (APTT) ≤ 1.5 × ULN.

### Exclusion criteria:

1. Previous exposure to any anti-PD-1 antibody, anti-PD-L1 antibody, HDAC inhibitor, or other medicaments, or previous treatment with a small-molecule anti-angiogenic therapy.
2. Treatment with any investigational drug within 4 weeks prior to the first dose of study treatment.
3. Treatment with the last dose of anti-tumor therapy (chemotherapy, targeted therapy, tumor immunotherapy, tumor embolization, etc.) within 3 weeks prior to the first dose.
4. Treatment with a radiotherapy within 4 weeks prior to the first dose administration.
5. Use of immunosuppressive drugs within 4 weeks prior to the first dose, excluding intranasal, inhaled, or other routes of local or physiological doses of systemic corticosteroids.
6. Exposure to toxicity that is caused by a previous anti-tumor therapy and has not recovered to ≤ Grade 1 per NCI CTCAE version 5.0., prior to the first dose.
7. Known symptomatic central nervous system metastases and/or carcinomatous meningitis.
8. Known acute or chronic active hepatitis B (HBsAg positive and HBV DNA ≥ 2000 IU/mL or ≥ 10⁴ copies/mL) or known acute or chronic active hepatitis C (HCV antibody positive and HCV RNA positive).
9. Uncontrolled arterial hypertension despite the administration of standard treatment (systolic blood pressure ≥ 150 mmHg or diastolic blood pressure ≥ 100 mmHg).
10. More than 3 loose stools per day at baseline, indicating susceptibility to poorly controlled colonic or small intestinal diseases.
11. Medical history of gastrointestinal perforation and/or fistula, peptic ulcer, intestinal obstruction (including incomplete intestinal obstruction requiring parenteral nutrition), extensive bowel resection (partial colectomy or extensive small bowel resection with chronic diarrhea), Crohn's disease, ulcerative colitis, intra-abdominal abscess, or chronic long-term diarrhea within the past 6 months. Implantation of intestinal stent.

### III. Test materials:

Sintilimab: Innovent Biologics (Suzhou) Co., Ltd. (Brand Name: Tyvyt^{®});
Chidamide: Shenzhen Chipscreen Biosciences Co., Ltd. (Brand Name: Epidaza^{®});
IBI305 (bevacizumab biosimilar): Innovent Biologics (Suzhou) Co., Ltd.

### III. Test protocol

A total of 48 patients were competitively enrolled and randomly divided into two groups: a CP dual-drug group and a CAP triple-drug group. The CAP triple-drug group consisted of 25 patients and was administered a triple-drug combination therapeutic regimen of sintilimab + chidamide + IBI305. The CP dual-drug group consisted of 23 patients and was administered a dual-drug combination therapeutic regimen of sintilimab + chidamide.

### Dosage regimen for CAP triple-drug group:

Sintilimab was administered at 200 mg via IV drip once every 3 weeks, on Day 1 of each cycle.

Chidamide was administered at 30 mg twice weekly via PO 30 minutes after meals, starting from Day 1 of Cycle 1, with an interval of no less than 3 days between doses (e.g., Monday and Thursday, Tuesday and Friday, Wednesday and Saturday, etc.). If chidamide was suspended from administration, it should be administered twice weekly starting from the time of resumption upon restarting the administration.

IBI305 was administered at 7.5 mg/kg via IV drip on Day 1 of each 3-week treatment cycle.

Sintilimab was first administered via IV drip over 60 (± 15) minutes; and after an interval of at least 5 minutes, IBI305 was administered at 7.5 mg/kg via IV drip. When any drug is temporarily or permanently discontinued, other drugs may still be administered for continued treatment.

### Dosage regimen for CP dual-drug group:

Sintilimab was administered at 200 mg via IV drip once every 3 weeks, on Day 1 of each cycle.

Chidamide was administered at 30 mg twice weekly via PO 30 minutes after meals, starting from Day 1 of Cycle 1, with an interval of no less than 3 days between doses (e.g., Monday and Thursday, Tuesday and Friday, Wednesday and Saturday, etc.). If chidamide was suspended from administration, it should be administered twice weekly starting from the time of resumption upon restarting the administration.

**Efficacy evaluation:** Based on RECIST v1.1, the 18-week progression-free survival (PFS), median PFS, ORR, partial response (PR), stable disease (SD), progressive disease (PD), neutrophils (NE), and DCR were evaluated in patients from both groups.

**Safety evaluation:** First 3 days of each cycle.

### IV. Test results

1. Efficacy endpoints included 18-week progression-free survival (18wPFS), objective response rate (ORR), partial response rate (PR), stable disease (SD), progressive disease (PD), neutrophils (NE), and disease control rate (DCR), etc.

As shown in Table 1, the median 18wPFS for patients in the CAP triple-drug group reached 66.7%, while that for patients in the CP dual-drug group was only 21.7%, showing a statistically significant difference (p = 0.0006). The efficacy of the CAP triple-drug regimen was significantly superior to that of the CP dual-drug regimen. Furthermore, compared to the CP dual-drug group, patients in the CAP triple-drug group demonstrated significantly superior outcomes in ORR, PR, PD, NE, and DCR (as shown in Table 1). The test results show that, compared to the CP dual-drug group, the CAP triple-drug group demonstrates unexpectedly superior therapeutic efficacy in the treatment of advanced microsatellite stability type colorectal cancer.

**Table 1 Comparison of efficacy between CAP triple-drug group and CP dual-drug group in the present invention**

| Efficacy endpoints | Sintilimab + chidamide + IBI305 (n = 25) | Sintilimab + chidamide (n = 23) |
|---|---|---|
| ORR | 11 (44.0%) | 3 (13.0%) p=0.027 |
| PR | 11 (44.0%) | 3 (13%) |
| SD | 7 (28.0%) | 6 (26.1%) |
| PD | 6 (24.0%) | 14 (60.9%) |
| NE | 1 (4.0%) | 0 |
| DCR | 18 (72.0%) | 9 (39.1%) p=0.045 |
| 18wPFS (%) | 66.7% | 21.7% p=0.0006 |

### 2. Median PFS

As shown in FIG. 1, the median progression-free survival (PFS) of the CP dual-drug group was approximately 1.6 months, while that of the CAP triple-drug group was about 7.35 months, demonstrating a statistically significant difference (p = 0.009) between the two. The CAP triple-drug group showed unexpectedly prolonged PFS in patients with MSS/MSI-L locally advanced unresectable or metastatic colorectal adenocarcinoma as compared to the CP dual-drug group. In summary, the triple-drug combination of chidamide + sintilimab + IBI305 significantly prolongs the progression-free survival in patients as compared to the dual-drug combination of chidamide + sintilimab. In addition, the patients in the triple-drug group demonstrates significant benefits in ORR, PR, PD, NE, and DCR.

Further, with reference to similar types of marketed medicaments, patients that have received the CAP triple-drug combination regimen of the present invention have achieved unexpected and encouraging therapeutic effects in terms of median progression-free survival (PFS), ORR, and the like (Table 2).

**Table 2 Comparison of efficacy between CAP triple-drug combination therapeutic regimen of the present invention and marketed drugs of the same Type**

| **Study** | **CAPability-01 (Phase 2)** | | **CONCUR (Asia Phase 3)** | **CORRECT (Global Phase 3)** | **FRESCO (China Phase 3)** | **FRESCO-2 (Global Phase 3)** | **TERRA (Asia Phase 3)** | **RECO USE (Global Phase 3)** |
|---|---|---|---|---|---|---|---|---|
| **Treatme nt regimen** | **Chidamide + sintilimab + bevacizumab** | **Chidamide + sintilimab** | **Regorafenib * Control placebo** | | **Fruquintinib * Control placebo** | | **TAS-102 * Control placebo** | |
| **Number of treatme nt lines** | ≥3 | ≥3 | ≥3 | ≥2* | ≥3 | ≥3 | ≥3 | ≥3 |
| **Sample Size** | 25 | 23 | 136 | 505 | 278 | 461 | 271 | 534 |
| **Median PFS (months )** | 7.35 | 1.6 | 3.2 | 1.9 | 3.7 | 3.7 | 2.0 | 2.0 |
| **ORR** | 44.0% | 13.0% | 4.4% | 1.0% | 4.7% | 1.5% | 1.1% | 1.6% |
| **Data source** | -- | | 2015Lan cet | 2013Lan cet | 2018JA MA | 2023Lance t | 2018JC O | 2015NE JM |

| | | | | | | | | |
|---|---|---|---|---|---|---|---|---|
| *In the CORRECT study, 16 patients in the regorafenib group had previously received only first-line treatment. | | | | | | | | |

The present invention has been described in detail above, with specific examples used herein to illustrate the principles and embodiments of the present invention. The descriptions of the above embodiments are intended solely to aid in understanding the method and core concepts of the present invention, including the best mode, and to enable any person skilled in the art to practice the present invention. It should be noted that a person of ordinary skill in the art may also make various improvements and modifications to the present invention without departing from the principles of the present invention, and these improvements and modifications shall be construed as also falling within the protection scope of the claims of the present invention. The scope of patent protection of the present invention is defined by the claims and may include other embodiments conceivable by a person skilled in the art. If these other embodiments include structural elements that do not differ from the literal expression of the claims, or if they include equivalent structural elements with no substantial differences from the literal expression of the claims, these other embodiments shall also fall within the scope of the claims.

## Claims

1. Use of chidamide or a derivative thereof in combination with a component (2) and a component (3) in third-line or higher-line prevention and/or treatment and/or amelioration of a locally advanced unresectable or metastatic colorectal cancer disease or disease progression, wherein
the derivative of chidamide is a pharmaceutically acceptable salt, crystal form, isomer, prodrug, or metabolite of chidamide;
the component (2) is selected from the group consisting of sintilimab, an antigen-binding fragment thereof, a variant thereof, and a biosimilar thereof; and
the component (3) is selected from the group consisting of bevacizumab, an antigen-binding fragment thereof, a variant thereof, and a biosimilar thereof.

2. Use of chidamide or a derivative thereof in manufacture of a combined medicament for third-line or higher-line prevention and/or treatment and/or amelioration of a locally advanced unresectable or metastatic colorectal cancer disease or disease progression,
wherein the combined medicament comprises chidamide or a derivative thereof and further comprises a component (2) and a component (3), wherein:
the derivative of chidamide is a pharmaceutically acceptable salt, crystal form, isomer, prodrug, or metabolite of chidamide;
the component (2) is selected from the group consisting of sintilimab, an antigen-binding fragment thereof, a variant thereof, and a biosimilar thereof; and
the component (3) is selected from the group consisting of bevacizumab, an antigen-binding fragment thereof, a variant thereof, and a biosimilar thereof.

3. The use according to claim 1 or 2, wherein:
the colorectal cancer is colorectal adenocarcinoma;
preferably, the colorectal adenocarcinoma is of a non-adenosquamous-mixed carcinoma type;
preferably, the colorectal cancer has at least previously undergone failed second-line and
higher-line standard treatment or intolerance to toxicity of second-line and higher-line standard treatment;
preferably, the standard treatment comprises fluorouracils, oxaliplatin, or irinotecan, the fluorouracils comprising 5-fluorouracil, capecitabine, tegafur, and the like;
preferably, the colorectal cancer is of a microsatellite stability (MSS) type or a low microsatellite instability (MSI-L) type;
preferably, the colorectal cancer is of a microsatellite stability (MSS) type;
preferably, the colorectal cancer is of a low microsatellite instability (MSI-L) type;
preferably, the chidamide or the derivative thereof is selected from the group consisting of chidamide, a pharmaceutically acceptable salt and a crystalline form thereof;
preferably, the chidamide or the derivative thereof is selected from the group consisting of chidamide, and crystal forms A and B thereof;
further preferably, the chidamide or the derivative thereof is selected from chidamide;
preferably, the component (2) is selected from the group consisting of sintilimab, an antigen-binding fragment thereof, and a biosimilar thereof;
further preferably, the component (2) is selected from sintilimab;
preferably, the component (3) is selected from the group consisting of bevacizumab, an antigen-binding fragment thereof, and a biosimilar thereof;
preferably, the component (3) is selected from the group consisting of bevacizumab,and a biosimilar thereof; and
more preferably, the component (3) is selected from the group consisting of bevacizumab and IBI305.

4. A pharmaceutical composition for use in third-line and higher-line prevention and/or treatment and/or amelioration of a locally advanced unresectable or metastatic colorectal cancer disease or disease progression, comprising a component (1), a component (2), and a component (3), wherein
the component (1) is selected from the group consisting of chidamide, a pharmaceutically acceptable salt, crystal form, isomer, prodrug, and metabolite thereof;
the component (2) is selected from the group consisting of sintilimab, an antigen-binding fragment thereof, a variant thereof, and a biosimilar thereof; and
the component (3) is selected from the group consisting of bevacizumab, an antigen-binding fragment thereof, a variant thereof, and a biosimilar thereof;
preferably, the colorectal cancer is of a microsatellite stability (MSS) type or a low microsatellite instability (MSI-L) type;
preferably, the colorectal cancer is of a microsatellite stability (MSS) type;
preferably, the colorectal cancer is of a low microsatellite instability (MSI-L) type; preferably, the colorectal cancer is colorectal adenocarcinoma;
preferably, the colorectal adenocarcinoma is of a non-adenosquamous-mixed carcinoma type;
preferably, the colorectal cancer has at least previously undergone failed second-line and higher-line standard treatment or intolerance to toxicity of second-line and higher-line standard treatment;
preferably, the standard treatment comprises fluorouracils, oxaliplatin, or irinotecan, the fluorouracils comprising 5-fluorouracil, capecitabine, tegafur, and the like;
preferably, the colorectal cancer has not been previously treated with a small-molecule anti-angiogenic medicament;
preferably, the component (1) is selected from chidamide or a pharmaceutically acceptable salt or crystal form thereof;
further preferably, the component (1) is selected from the group consisting of chidamide, and crystal forms A and B thereof;
further preferably, the component (1) is selected from chidamide;
preferably, the component (2) is selected from the group consisting of sintilimab, an antigen-binding fragment thereof, and a biosimilar thereof;
further preferably, the component (2) is selected from sintilimab;
preferably, the component (3) is selected from the group consisting of bevacizumab, an antigen-binding fragment thereof, and a biosimilar thereof;
further preferably, the component (3) is selected from the group consisting of bevacizumab and a biosimilar thereof; and
more preferably, the component (3) is selected from the group consisting of bevacizumab and IBI305.

5. The pharmaceutical composition according to claim 4 for use in third-line and higher-line prevention and/or treatment and/or amelioration of a locally advanced unresectable or metastatic colorectal cancer disease or disease progression, wherein
preferably, the colorectal cancer is of a microsatellite stability (MSS) type or a low microsatellite instability (MSI-L) type;
preferably, the colorectal cancer is of a microsatellite stability (MSS) type;
preferably, the colorectal cancer is of a low microsatellite instability (MSI-L) type; preferably, the colorectal cancer is colorectal adenocarcinoma;
preferably, the colorectal adenocarcinoma is of a non-adenosquamous-mixed carcinoma type;
preferably, the colorectal cancer has at least previously undergone failed second-line and higher-line standard treatment or intolerance to toxicity of second-line and higher-line standard treatment;
preferably, the standard treatment comprises fluorouracils, oxaliplatin, or irinotecan, the fluorouracils comprising 5-fluorouracil, capecitabine, tegafur, and the like.

6. A kit, comprising the pharmaceutical composition according to claim 4.

7. The kit according to claim 6, wherein the component (1), the component (2), and the component (3) are unit preparations having the same or different strengths, and the component (1), the component (2), and the component (3) are placed in the same container or in separate containers respectively.

8. The kit according to claim 6 or 7, wherein the component (1) is a gastrointestinal dosage form, preferably an oral preparation; and the component (2) and the component (3) are parenteral dosage forms, preferably injectable preparations.

9. The kit according to claim 6 or 7, wherein the component (1) is chidamide, with a strength of 5 mg/tablet; the component (2) is sintilimab, with a strength of 100 mg/10 ml; and the component (3) is IBI305 or bevacizumab, each with a strength of 100 mg/4 ml or 400 mg/16 ml.

10. A method for third-line and higher-line prevention and/or treatment and/or amelioration of a locally advanced unresectable or metastatic colorectal cancer, comprising administering to a patient in need thereof a prophylactically and/or therapeutically and/or amelioratively effective amount of the pharmaceutical composition according to claim 4, wherein
preferably, the colorectal cancer is of a microsatellite stability (MSS) type or a low microsatellite instability (MSI-L) type;
preferably, the colorectal cancer is of a microsatellite stability (MSS) type;
preferably, the colorectal cancer is of a low microsatellite instability (MSI-L) type; preferably, the colorectal cancer is colorectal adenocarcinoma;
preferably, the colorectal adenocarcinoma is of a non-adenosquamous-mixed carcinoma type;
preferably, the colorectal cancer has at least previously undergone failed second-line and higher-line standard treatment or intolerance to toxicity of second-line and higher-line standard treatment;
preferably, the standard treatment comprises fluorouracils, oxaliplatin, or irinotecan, the fluorouracils comprising 5-fluorouracil, capecitabine, tegafur, and the like.
